# EUROPÄISCHE PATENTSCHRIFT

(11) **EP 1 220 700 B1**
(45) Veröffentlichungstag und Bekanntmachung des Hinweises auf die Patenterteilung: **09.04.2003**
(21) Anmeldenummer: 00982968.0
(22) Anmeldetag: 06.10.2000
(51) Int. Cl.: A61M 15/00

(54) **Atemzugskontrolliertes Inhalationsgerät für Trockenpulver**
Breathing-controlled inhalation device for dry powder
Inhalateur de respiration controlée pour poudre

(30) Priorität: 06.10.1999 DE 19948289; 06.06.2000 DE 10027631
(43) Veröffentlichungstag der Anmeldung: 10.07.2002
(73) Patentinhaber: Eckardt, Angela, 01309 Dresden (DE); Frydling, Ofer, 01189 Dresden (DE); Goldemann, Raul, 71700 Modlin (IL)
(72) Erfinder: GOLDEMANN, Raul, 71700 Modlin (DE)
(74) Vertreter: Hudler, Frank, Dipl.-Ing.
(86) Internationale Anmeldenummer: DE0003527
(87) Internationale Veröffentlichungsnummer: WO01024857

(56) Entgegenhaltungen:
- EP-A- 0 938 907
- WO-A-98/34661
- WO-A-99/33505
- DE-A- 4 340 768
- US-A- 5 699 789

## Beschreibung

Die Erfindung betrifft ein atemzugskontrolliertes Inhalationsgerät für Trockenpulver, insbesondere von mit Medikamenten vermischtem Trockenpulver, bestehend aus einem Gehäuse mit einem Mundstück, einer im Gehäuse befindlichen Vorratskammer für das Trockenpulver, sowie einer Luftführungseinheit zwischen einem Übergabebereich für das Trockenpulver und dem Mundstück, wobei die Vorratskammer mit einem Dosierförderer gekoppelt ist, der in den Übergabebereich derart eintaucht, dass unmittelbar am Anfang der Luftführungseinheit eine vorgegebenen Menge an Trockenpulver positionierbar ist und dass die anschließende Luftführungseinheit einen Strömungskanal aufweist, der abwechselnd mit Verengungen und jeweils nachfolgenden Erweiterungen versehen ist, wobei die Luftführungseinkeit aus einem im wesenflichen zylindrischen Grundkörper besteht.

Zur Behandlung von Atemwegserkrankungen ist die Zuführung entsprechender Wirkstoffe auf dem Wege der Inhalation seit langem eine der primären Methoden. Die dabei - oft auch als transporttaugliche Ausgestaltungen - verwendeten Geräte erlangen diesbezüglich durch die steigende Zahl der Atemwegserkrankungen während der letzten Jahre immer größere Bedeutung. Vor allem atemluftkontrollierte Geräte, die eine schonende Alternative zu treibgasgesteuerten Geräten darstellen, kommen vermehrt zum Einsatz, da sie nicht den unangenehmen Effekt einer Kältereizung verursachen.

Aus der DE 40 04 904 A1 ist eine Vorrichtung dieser Art bekannt, bei der der Wirkstoff an der Peripherie einer Dosiertrommel bereitgehalten und radial nachgestellt wird. Das Innere der Dosiertrommel enthält eine Steuervorrichtung zum aktiven Ausbringen der abgeteilten Inhaliermenge aus radial auswärts gerichteten, offenen Dosierausnehmungen. Dazu werden den Boden der winkelgleich verteilt angeordneten Dosierausnehmungen bildende Schieber zentral kurvengesteuert. Da diese Steuermechanik zudem von einer eine Längsseite der Vorrichtung nahezu voll einnehmenden Betätigungstaste ausgelöst wird, ist der diesbezügliche Aufwand erheblich. Außerdem sind die dosierten Arzneistoffe durch eine Zwangsentleerung der Dosierausnehmung addierbar, es kann daher zu einer gefährlichen überdosierung kommen. Der Raum, der für die Vorratskammer verbleibt, ist volumenmäßig nur ein Bruchteil der im Taschenformat ausgebildeten Vorrichtung.

Die DE 198 25 434 A1 beschreibt ein Inhalationsgerät, bei welchem die Dosiervorrichtung vor der Einnahme des Medikaments vorgespannt wird und in diesem Zustand an einem durch das Einatmen bewegbaren Anschlag gehalten wird. Mit dem Einatmen wird die Dosiervorrichtung freigesetzt und beschleunigt, wobei die beschleunigte Bewegung abrupt unterbrochen wird, indem das Anschlagstück der Dosiervorrichtung an das Gehäuse bzw. den Gehäuseboden anschlägt. Diese plötzliche Unterbrechung der Drehbewegung der Dosiervorrichtung hat zur Folge, dass das pulverförmige Medikament mit hoher Geschwindigkeit aus der Dosierkavität herausgeschleudert und weiträumig im Luftkanal verteilt wird. Auch dieser Aufbau ist relativ kompliziert und weist darüber hinaus den Mangel einer nichtvorhandenen strömungsorientierten Luftführung auf. Der Luftkanal ist geradlinig aufgebaut und ermöglicht keinerlei Zirkulation oder Verwirbelung der zu inhalierenden Stoffe.

Schließlich beschreibt die DE 43 40 768 A1 eine Vorrichtung zum Inhalieren pulverförmiger Stoffe, wobei eine spezielle Wirbelkammer in schneckenförmiger Ausgestaltung vorgesehen ist. Auch hier ist der im Übrigen komplizierte Konstruktionsaufbau nachteilig. Des weiteren ermöglicht die Wirbelkammer zwar eine gewisse Gleichmäßigkeit der Verteilung des Pulvers. Indess führt die schneckenförmige Windung der Wirbelkammer verstärkt zu Reibungs- und Widerstandspunkten, die einem vollständigen Durchzug der Partikel hinderlich sind.

Weiterhin geht aus der US-A-5,699,789, die zur Formulierung des Oberbegriffs des Anspruchs 1 herangezogen werden ist ein Inhalator für Trockenpulver hervor, bei dem ein Dosierförderer vorgesehen ist, der aus einem in den Strömungskanal hineinragenden Vorratsbehälter eine vorgegebene Menge an Trockenpulver entnimmt und innerhalb der Luftführungseinheit positioniert. Die den Strömungskanal passierende Lift muss sich um den Vorratsbehälter und um eine in der Nähe der Auslassöffnung befindliche Nase herumbewegen. Dadurch wird eine gewisse Verwirbelung der Luft erreicht, jedoch kann nicht sichergestellt werden, dass sämtliches zugeführtes Trockenpulver homogen verteilt durch die Auslassöffnung transportiert wird.

In der EP-A-938907 wird eine Inhalationsvorrichtung für Trockenpulver beschrieben, bei der wechselweise in den Strömungskanal hineinragende Platten vorgesehen sind, um eine Verwirbelung der Luft zu erreichen. Bei dieser Vorrichtung kann infolge der vielen Toträume nicht gesichert werden, dass die gesamte zudosierte Pulvermenge beim Inhalieren heraustransportiert wird.

Eine ähnliche Inhalationsvorrichtung geht aus der WO-A-9933505 hervor. Auch hier können Pulverrückstände in der Vorrichtung verbleiben, welche die Dosierung verfälschen.

Der Erfindung liegt damit die Aufgabe zugrunde, ein atemzugskontrolliertes Inhalationsgerät der eingangs genannten Art zu schaffen, das infolge eines einfachen Aufbaus mit wenigen Einzelteilen kostengünstig herstellbar ist, eine geringe Baugröße aufweist, mit einem Doppeldosierschutz ausgestattet ist und mit dem eine vollständige und gleichmäßige Verteilung des Trockenpulvers während des Inhalationsvorganges erreicht werden kann. Weiterhin soll ein Verfahren geschaffen werden, welches eine vollständige und gleichmaßige lungengängige Verteilung des zu inhalierenden Trockenpulvers in der Atemluft ermöglicht.

Die der Erfindung zugrundeliegende Aufgabenstellung wird dadurch gelöst, dass der Grundkörper wechselweise mit kugelkalottenähnlichen Einbuchtungen versehen ist, die sich von jeweils gegenüberliegenden Wandungen des Grundkörpers in den Strömungskanal der Luft erstrecken, und dass der Strömungskanal einen in Gebrauchslage des Inhalationsgerätes ansteigenden, sich an den Übergabe bereich auschliessen den Einzugsbereich aufweist. Die so ausgestaltete Luftführungseinheit erlaubt eine sehr effektive und vollständige Verteilung des Trockenpulvers während des Inhalationsvorganges, da die angesaugte Luft zirkuliert und die aufsteigenden Partikel optimal miteinander vermischt werden können.

Durch diese besondere Ausbildung des Einzugsbereiches kann das Trockenpulver im Bedarfsfall direkt in den Strömungskanal gelangen, und von dort unmittelbar angesaugt werden. Hierdurch wird das Risiko des Eindringens von Feuchtigkeit oder eines ungewollten anteiligen Verlustes der Portionierung während der Inhalation verringert.

In einer weiteren günstigen Ausführungsform der Erfindung ist das Gehäuse mit einem Lufteinlass versehen, der stromab gegenüber dem Einzugsbereich angeordnet ist. Die Öffnung der Vorrichtung in Form des Lufteinlasses ermöglicht ein verstärktes und Restluft unabhängiges Ansaugen der Partikel.

Ferner sieht eine weitere Ausführungsform vor, dass die Vorratskammer gegenüber dem Einzugsbereich verriegelt ist, indem der Dosierförderer als flacher Schieber ausgeführt ist, der eine quer verlaufende Dosierbohrung zur Aufnahme des Trockenpulvers aufweist, wobei sich die Dosierbohrung im geschlossenen Zustand der Vorratskammer in dieser befindet und in Dosierposition unmittelbar vor dem Einzugsbereich der Luftführungseinheit und dass der Schieber die Vorratskammer im wesentlichen ständig verschlossen hält. Auf diese Weise kann ein Doppeldosierschutz sichergestellt werden, da pro Inhalationsvorgang nur die in der Dosierbohrung befindliche Menge an Trockenpulver zur Verfügung steht. Wird der Inhalationsvorgang abgebrochen oder unterbrochen, so wird das restliche in der Dosierbohrung befindliche Trockenpulver in die Vorratskammer zurückbefördert, oder fällt aus dem Einzugsbereich heraus und steht damit bei einem weiteren Inhalationsvorgang nicht mehr zur Verfügung.

Eine günstige Ausführungsform sieht hierbei vor, dass der Schieber federbelastet in einer Ausgangsposition gehalten wird, in der die Vorratskammer verschlossen ist und dass der Schieber entgegen einer Federkraft in die Dosierposition bewegbar ist. Ein federgesteuerter Bewegungsmechanismus gewährleistet, dass ein versehentlicher Austritt, ein ungewolltes Eindringen von Feuchtigkeit oder ein nicht vorgesehenes Betätigen der Vorrichtung nahezu ausgeschlossen werden. Außerdem liegt ein wesentlicher Vorteil dieser Ausgestaltung darin, dass wegen der Einfachheit des funktionalen Aufbaus, abgesehen von der nötigen Rückstellfeder, nur ein bewegtes Teil (Dosierförderer) benötigt wird, was wiederum die ständige Einsatzbereitschaft des Gerätes garantiert und das Risiko etwaiger Fehlfunktionen minimiert.

Die Luftführungseinheit kann einstückig oder mehrstückig ausgebildet sein, wobei sich Glas oder Kunststoff als kostengünstige Materialien als besonders geeignet erwiesen haben. Selbstverständlich können auch andere geeignete Materialien, wie Metalle, für die Herstellung der Luftführungseinheit verwendet werden.

Durch die Anreicherung der Partikelströme mit kinetischer Energie wird deren Bewegungs- und Verteilungsleistung erhöht und ein etwaiger schwerkraftbedingter Energieverlust kompensiert, was den Verwirbelungseffekt insgesamt verbessert.

Die Erfindung soll nachfolgend anhand eines Ausführungsbeispiels näher erläutert werden.

Dabei zeigen:
- Fig. 1a: eine Vorderansicht eines erfindungsgemäßen Inhalationsgerätes im Ruhezustand mit geöffnetem Mundstück;
- Fig. 1b: eine Schnittdarstellung entlang der Linie A-A nach Fig. 1a;
- Fig. 1c: eine Schnittdarstellung entlang der Linie B-B nach Fig. 2b;
- Fig. 1d: eine perspektivische Darstellung des erfindungsgemäßen Inhalationsgerätes nach Fig. 1a - 1c;
- Fig. 2a: eine Vorderansicht eines erfindungsgemäßen Inhalationsgerätes im Ruhezustand mit geschlossenem Mundstück;
- Fig. 2b: eine Schnittdarstellung entlang der Linie A-A nach Fig. 2a;
- Fig. 2c: eine Schnittdarstellung entlang der Linie B-B nach Fig. 2b;
- Fig. 2d: eine perspektivische Darstellung des erfindungsgemäßen Inhalationsgerätes nach Fig. 2a - 2c;
- Fig. 3a: eine Vorderansicht eines erfindungsgemäßen Inhalationsgerätes im inhalationsbereiten Zustand mit geöffnetem Mundstück;
- Fig. 3b: eine Schnittdarstellung entlang der Linie A-A nach Fig. 3a;
- Fig. 3c: eine Schnittdarstellung entlang der Linie B-B nach Fig. 3b;
- Fig. 3d: eine perspektivische Darstellung des erfindungsgemäßen Inhalationsgerätes nach Fig. 3a - 3c;
- Fig. 4a: eine Vorderansicht eines erfindungsgemäßen Inhalationsgerätes im inhalationsbereiten Zustand mit geschlossenem Mundstück;
- Fig. 4b: eine Schnittdarstellung entlang der Linie A-A nach Fig. 4a;
- Fig. 4c: eine Schnittdarstellung entlang der Linie B-B nach Fig. 4b;
- Fig. 4d: eine perspektivische Darstellung des erfindungsgemäßen Inhalationsgerätes nach Fig. 4a - 4c;
- Fig. 5: eine Explosionsdarstellung der Einzelteile des erfindungsgemäßen atmungskontrollierten Inhalationsgerätes.

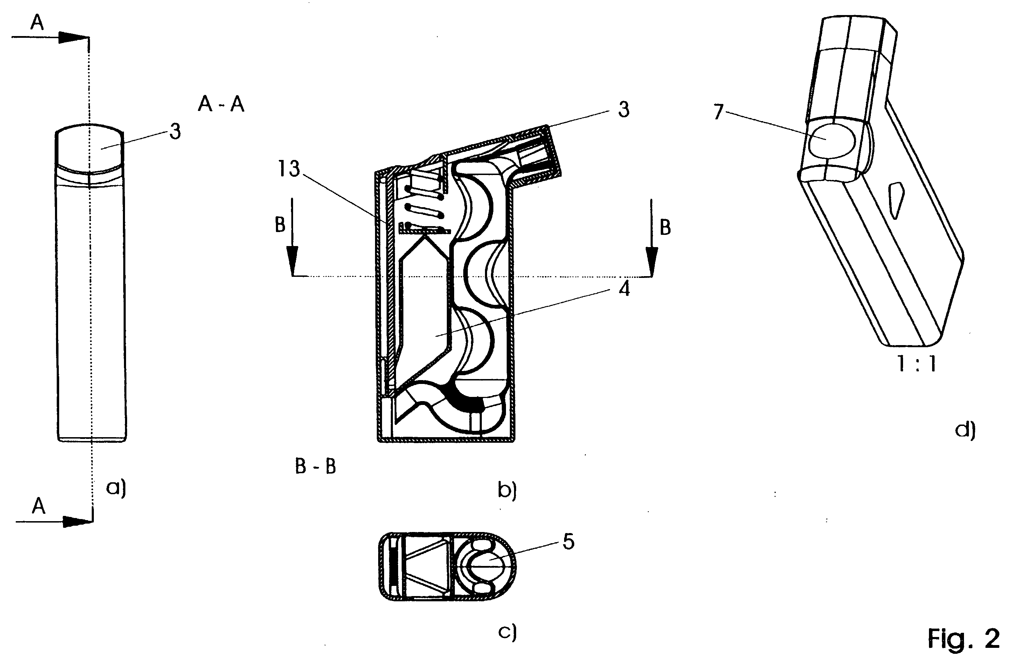

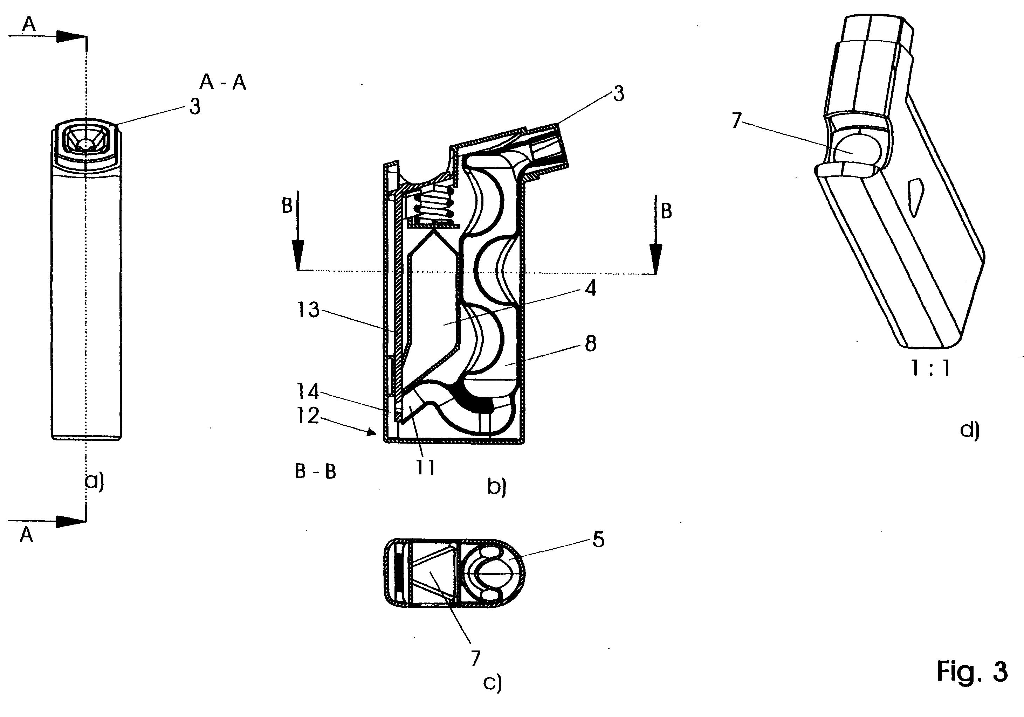

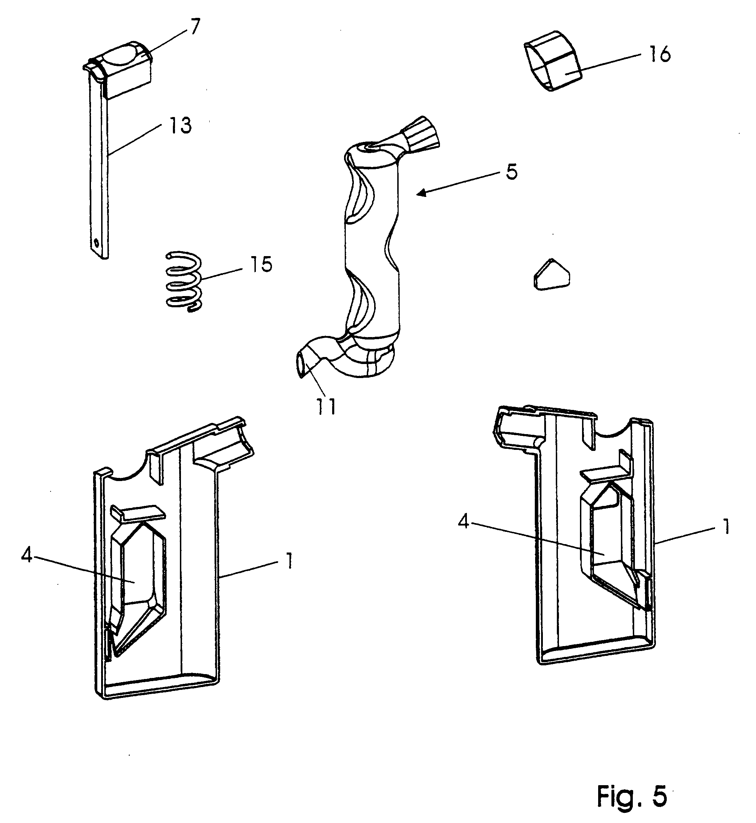

Wie in den Fig. 2a-d und 3a-d gezeigt, wird die Vorratskammer 4 sowohl im unbenutzten als auch im benutzen Zustand durch einen als Schieber 13 (gesondert gezeigt in Fig. 5) ausgestalteten Dosierförderer 7 (ebenfalls gesondert dargestellt in Fig. 5) im wesentlichen ständig verschlossen gehalten. Ein Eindringen von externer Feuchtigkeit oder Verunreinigungen kann somit in Hinblick auf das in der Vorratskammer befindliche Trockenpulver nahezu ausgeschlossen werden.

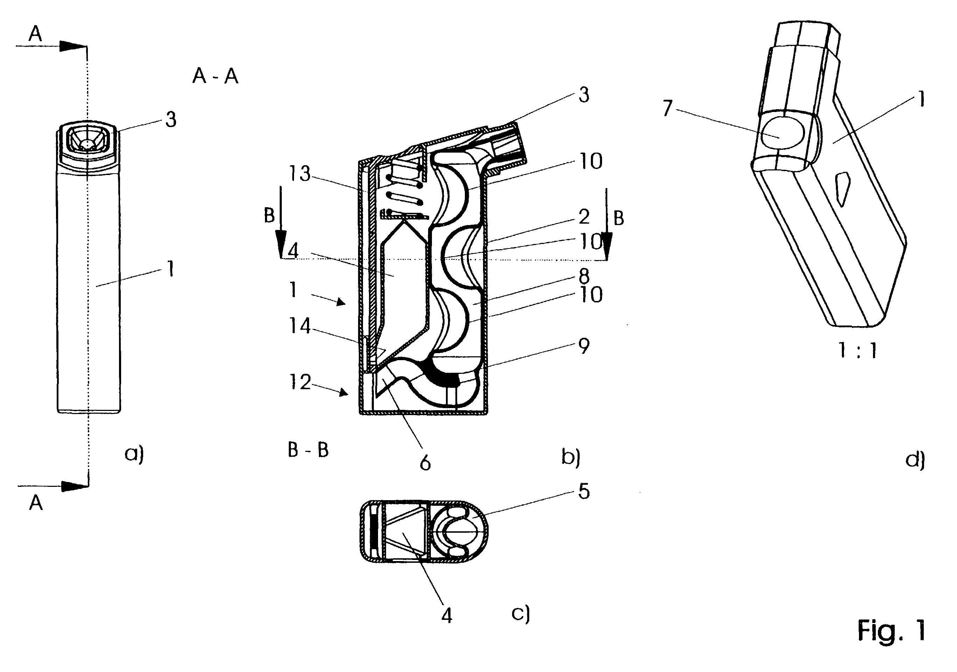

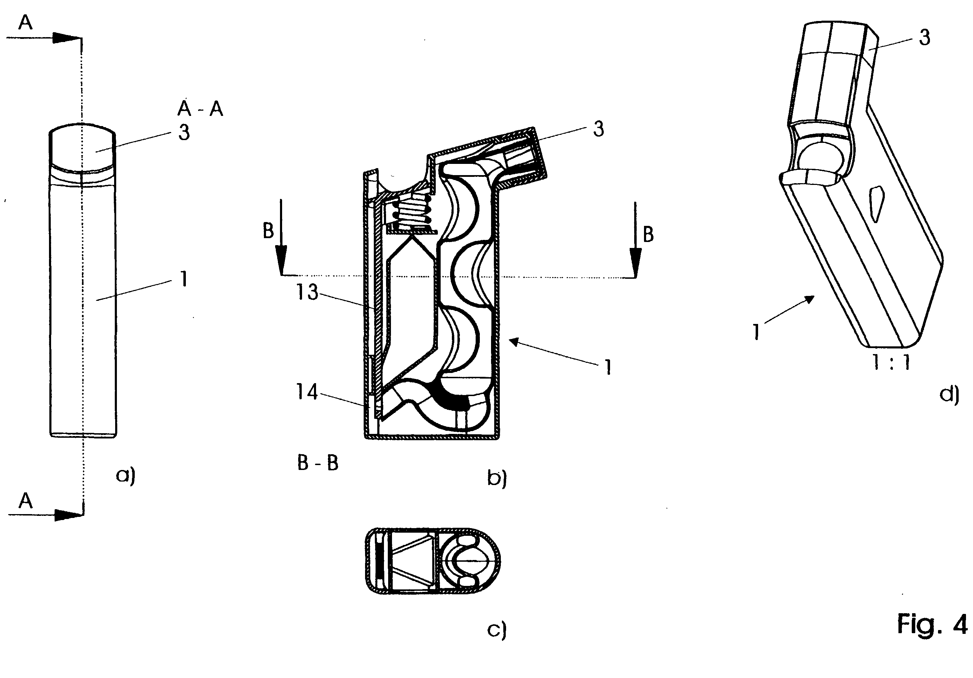

Der Dosierförderer 7 weist eine quer verlaufende Dosierbohrung 14 (gezeigt in Fig. 1b) zur Aufnahme des Trockenpulvers auf. Vor dem Beginn des Inhalationsvorganges befindet sich die Dosierbohrung 14 in der Vorratskammer 4. Eine Kappe 16 (gesondert dargestellt in Fig. 5) schützt das Mundstück 3 hygienisch. Das Inhalationsgerät 1 wird, nachdem die Kappe 16 entfernt wurde (Fig. 1a und b), senkrecht mit dem Mundstück 3 nach oben vom Mund entfernt gehalten. Nachdem der Mutzer zunächst unter Vermeidung eines Kontakts mit dem Mundstück - d.h. ohne in das Mundstück 3 zu blasen - tief ausgeatmet hat, umschließt er daraufhin das Mundstück 3 mit den Lippen. Um den Inhalationsvorgang zu starten und den Dosierförderer 7 in die Dosierposition zu versetzen, wird dieser entgegen einer durch eine in Fig. 5 gesondert dargestellten Feder 15 erzeugten Kraft nach unten gedrückt (Fig. 3b und 3d, sowie Fig. 4b und 4d).

Die Dosierbohrung 14 befindet sich nun unmittelbar vor einem ansteigenden Einzugsbereich 11 der Luftführungseinheit 5 (Fig. 4b und 3b). Dadurch, dass die Vorratskammer 4 mit dem Dosierförderer 7 derart gekoppelt ist, dass dieser in einen Übergabebereich 6 vor dem Einzugsbereich eintaucht und die vorgegebenen Menge an Trockenpulver unmittelbar am Anfang der Luftführungseinheit 5 positionierbar ist, wird gewährleistet, dass es nicht zu einem ungewollten Verlust der Portionierung vor oder während der Inhalation kommt.

Der Nutzer atmet nun so tief wie möglich durch den Mund ein.

Durch einen im Gehäuse 2 befindlichen Lufteinlass 12, der - wie in Fig. 3b dargestellt - stromab gegenüber dem Einzugsbereich 11 angeordnet ist, wird Luft angesaugt und es entsteht ein Luftetrom innerhalb des Inhalationsgerätes 1. Dieser trägt die Partikel des Trockenpulvers über die Dosierbohrung 14 durch den Übergabe- und Einzugsbereich 6, 11 und schließlich durch die Luftführungseinheit 5 bis es zu einem Austritt der Partikel durch das Mundstück 3 direkt in die Atemwege des Nutzers kommt.

Wird der Inhalationsvorgang abgebrochen oder unterbrochen, so wird das restliche in der Dosierbohrung 14 befindliche Trockenpulver in die Vorratskammer 4 zurückbefördert, indem die Feder den Dosierförderer in die Ausgangslage versetzt, oder fällt aus dem Einzugsbereich heraus und steht damit bei einem weiteren Inhalationsvorgang nicht mehr zur Verfügung. Auf diese Weise wird die Gefahr einer Doppeldosierung vermieden.

Die infolge der Atmung angesaugte Atemluft wird derart durch die - in Fig. 5 gesondert dargestellte - Luftführungseinheit geführt, dass abwechselnd eine Beschleunigung und ein nachfolgendes Abbremsen des Luftstromes bei gleichzeitiger Verwirbelung und Änderung der Strömungsrichtung erfolgt.

Ermöglicht wird dies dadurch, dass die Luftführungseinheit 5 aus einem im wesentlichen zylindrischen Grundkörper 9 besteht, der wechselweise mit kugelkalottenähnlichen Einbuchtungen 10 versehen ist, die sich von jeweils gegenüberliegenden Wandungen des Grundkörpers 9 in den Strömungskanal 8 der Luft erstrecken.

Die Luftführungseinheit 5 kann einstückig oder mehrstückig ausgebildet sein, wobei sich Glas oder Kunststoff als kostengünstige Materialien als besonders geeignet erwiesen haben. Selbstverständlich können auch andere geeignete Materialien, beispielsweise Metalle für die Herstellung der Luftführungseinheit verwendet werden.

Die Beschleunigung der Luftströmung wird dabei durch Querschnittsverengungen in der Luftführungseinheit 5 vorgenommen, die sich abwechselnd auf jeweils gegenüberliegenden Seiten in der Luftführungseinheit 5 befinden. Die so ausgestaltete Luftführungseinheit 5 erlaubt eine sehr effektive und vollständige Verteilung des Trockenpulvers während des Inhalationsvorganges, da die angesaugte Luft zirkuliert und die aufsteigenden Partikel optimal miteinander vermischt werden können.

Durch die Anreicherung der Partikelströme mit kinetischer Energie wird deren Bewegungs- und Verteilungsleistung erhöht und ein etwaiger schwerkraftbedingter Energieverlust kompensiert, was den Verwirbelungseffekt insgesamt verbessert.

### Bezugszeichenliste

- 1: Inhalationsgerät
- 2: Gehäuse
- 3: Mundstück
- 4: Vorratskammer
- 5: Luftführungseinheit
- 6: Übergabebereich
- 7: Dosierförderer
- 8: Strömungskanal
- 9: Grundkörper
- 10: Einbuchtung
- 11: Einzugsbereich
- 12: Lufteinlaß
- 13: Schieber
- 14: Dosierbohrung
- 15: Feder
- 16: Kappe

## Patentansprüche

1. Atemzugskontrolliertes Inhalationsgerät für Trockenpulver, insbesondere von mit Medikamenten vermischtem Trockenpulver, bestehend aus einem Gehäuse mit einem Mundstück (3), einer im Gehäuse (2) befindlichen Vorratskammer (4) für das Trockenpulver, sowie einer Luftführungseinheit (5) zwischen einem Übergabebereich für das Trockenpulver und dem Mundstück (3), wobei die Vorratskammer (4) mit einem Dosierförderer (7) gekoppelt ist, der in den Übergabebereich (6) derart eintaucht, dass unmittelbar am Anfang der Luftführungseinheit (5) eine vorgegebenen Menge an Trockenpulver positionierbar ist und dass die anschließende Luftführungseinheit (5) einen Strömungskanal (8) aufweist, der abwechselnd mit Verengungen und jeweils nachfolgenden Erweiterungen versehen ist, wobei die Luftführungseinheit (5) aus einem im wesentlichen zylindrischen Grundkörper (9) besteht, **dadurch gekennzeichnet, dass** der Grundkörper (9) wechselweise mit kugelkalottenähnlichen Einbuchtungen (10) versehen ist, die sich von jeweils gegenüberliegenden Wandungen des Grundkörpers (9) in den Strömungskanal (8) der Luft erstrecken, und dass der Strömungskanal (8) einen in Gebrauchslage des Inhalationsgerätes (1) ansteigenden, sich an den übergabebereich (6) ausschliessenden Einzugsbereich (11) aufweist.

2. Atemzugskontrolliertes Inhalationsgerät nach Anspruch 1, **dadurch gekennzeichnet, dass** das Gehäuse (2) mit einem Lufteinlass (12) versehen ist, der stromab gegenüber dem Einzugsbereich (11) angeordnet ist.

3. Atemzugskontrolliertes Inhalationsgerät nach Anspruch 1 und 2 **dadurch gekennzeichnet, dass** die Vorratskammer (4) gegenüber dem Einzugsbereich (11) verriegelt ist, indem der Dosierförderer (7) als flacher Schieber (13) ausgeführt ist, der eine quer verlaufende Dosierbohrung (14) zur Aufnahme des Trockenpulvers aufweist, wobei sich die Dosierbohrung (14) im geschlossenen Zustand der Vorratskammer (4) in dieser befindet und in Dosierposition unmittelbar vor dem Einzugsbereich (11) der Luftführungseinheit (5) und dass der Schieber (13) die Vorratskammer (4) im wesentlichen ständig verschlossen hält.

4. Atemzugskontrolliertes Inhalationsgerät nach Anspruch3, **dadurch gekennzeichnet, dass** der Schieber (13) federbelastet in einer Ausgangsposition gehalten wird, in der die Vorratskammer (4) verschlossen ist und dass der Schieber (13) entgegen einer Federkraft in die Dosierposition bewegbar ist.

5. Atemzugskontrolliertes Inhalationsgerät nach einem der Ansprüche 1 bis4, **dadurch gekennzeichnet, dass** die Luftführungseinheit (5) ein- oder mehrstückig ausgebildet ist.

6. Atemzugskontrolliertes Inhalationsgerät nach einem der Ansprüche 1 bis5, **dadurch gekennzeichnet, dass** die Luftführungseinheit (5) aus Glas, Kunststoff oder einem anderen geeigneten Material gefertigt ist.

## Claims

1. Breathing-controlled inhalation device for dry powder, particularly of dry powder mixed with medicines, consisting of a casing with a mouthpiece (3), a reservoir (4) for the dry powder located in the casing (2), as well as an air guiding unit (5) between a transfer area for the dry powder and the mouthpiece(3), whereby the reservoir (4) is connected to a dosing conveyor (7) which dips in the transfer area (6) in such a way that a predetermined amount of dry powder can be positioned directly at the beginning of the air guiding unit (5) and that the attached air guiding unit (5) has an air-flow channel (8) which is provided alternately with narrowings and subsequent enlargements, **characterized in that** air guiding unit (5) consists of an essentially cylindrical central component (9), **characterized in that** it is provided alternately with semi-spherical indentations (10) which reach from opposite walls of the central component (9) into the air-flow channel (8), **in that** the air-flow channel (8) has a rising inhalation area (11) in the application position of the inhalation device (1), immediately following the transfer area (6).

2. Breathing-controlled inhalation device according to claim 1, **characterized in that** the casing (2) is equipped with an air inlet (12), which is arranged downwards opposite the inhalation area (11).

3. Breathing-controlled inhalation device according to claim 1, **characterized in that** the reservoir (4) opposite the inhalation area (11) is locked by means of the dosing conveyor (7) being designed as a flat slide (13), which has a laterally positioned dosing drill hole (14) for taking in the dry powder, **in that** the dosing drill hole (14) is located inside the reservoir (4) in its closed state and in dosing position immediately in front of the inhalation area (11) of the air guiding unit (5) and that the slide (13) generally keeps the reservoir (4) shut.

4. Breathing-controlled inhalation device according to claim 3, **characterized in that** the slide is held in a spring-loaded start position, in which the reservoir (4) is locked and that the slide (13) is moveable against a spring resistance in the dosing position.

5. Breathing-controlled inhalation device according to one of the claims 1 to 4, **characterized in that** the air guiding unit (5) has a single-part or multi-part design.

6. Breathing-controlled inhalation device according to one of the claims 1 to 5, **characterized in that** the air guiding unit (5) is manufactured from glass, plastic or another suitable material.

## Revendications

1. L'appareil d'inhalation pour poudre sèche contrôlé par l'aspiration, en particulier de la poudre sèche mélangée avec des médicaments, composé d'un boîtier avec une embouchure (3), d'un réservoir (4) se trouvant dans le boîtier (2) pour la poudre sèche, ainsi que d'une unité de ventilation (5) entre la zone de transfert de la poudre sèche et l'embouchure (3), ce qui le réservoir (4) est relié avec un transporteur doseur (7) plongant dans la zone de transfert (6) de telle sorte qu'une quantité de poudre sèche fixée au préalable est positionnable immédiatement au début de l'unité de ventilation (5) et que l'unité de ventilation (5) raccordée a un canal d'écoulement (8), qui est doté de façon alternante de constrictions et d'élargissement, cependant, ce qui l'unité de ventilation (5) est constituée d'un corps de base (9) essentiellement cylindrique, caractérisé du fait que ce corps de base (9) est doté alternativement avec des baies (10) s'étendant des parois opposées correspondantes du corps de base (9) au canal d'écoulement d'air (8), et que en position d'usage de l'appareil d'inhalation (1) ce canal d'écoulement d'air (8) montre une zone d'entrée (11) grandissante rattachée a la zone de transfert (6).

2. L'appareil d'inhalation contrôlé par l'aspiration selon la Revendication 1, caractérisé du fait que le boîtier (2) a une admission gazeuse (12), se trouvant en aval en face de la zone d'entrée (11).

3. L'appareil d'inhalation contrôlé par l'aspiration selon la Revendication 1, caractérisé du fait que le réservoir (4) en face de la zone d'entrée (11) est réglé du fait que le transporteur doseur (7) est un coulisseau plat (13) ayant un trou de dosage courant pour l'admission de poudre sèche, ce qui le trou de dosage (14) se trouve dans le réservoir (4) dans un état ferme du réservoir (4) et devant la zone d'entrée (11) de l'unité de ventilation (5) en position de dosage, et que le coulisseau (13) arrête le réservoir (4) essentiellement toujours fermé.

4. L'appareil d'inhalation contrôlé par l'aspiration selon la Revendication 3, caractérisé du fait que le coulisseau (13) est arrêté par un ressort en la position de départ dans laquelle le réservoir (4) est fermé et que le coulisseau (13) bouge contre la force de ressort en position de dosage.

5. L'appareil d'inhalation contrôlé par l'aspiration selon les Revendications 1 à 4, caractérisé du fait que l'unité de ventilation (5) est constituée d'un ou plusieurs parties.

6. L'appareil d'inhalation contrôlé par l'aspiration selon les Revendications 1 à 5, caractérisé du fait que l'unité de ventilation (5) est fabriquée en verre, plastique où bien autre matériel spécifique.
